# EUROPEAN PATENT SPECIFICATION

(11) **EP 1 999 441 B1**
(45) Date of publication and mention of the grant of the patent: **19.06.2013**
(21) Application number: 07727107.0
(22) Date of filing: 20.03.2007
(51) Int. Cl.: A61M 5/28, G01F 23/26, A61M 5/145, G01F 11/02, A61M 5/178

(54) **DETERMINATION OF CARTRIDGE CONTENT BY CAPACITIVE MEANS**
BESTIMMEN EINES PATRONENINHALTS DURCH KAPAZITIVE MITTEL
DÉTERMINATION DU CONTENU DE CARTOUCHES PAR DES MOYENS CAPACITIFS

(30) Priority: 20.03.2006 EP 06005602
(43) Date of publication of application: 10.12.2008
(73) Proprietor: Novo Nordisk A/S, 2880 Bagsværd (DK)
(72) Inventor: LARSEN, André, DK-2791 Dragør (DK)
(86) International application number: PCT/EP2007/052630
(87) International publication number: WO 2007/107558

(56) References cited:
- WO-A-00/37129
- DE-A1- 3 923 079
- US-A- 5 135 485
- US-A- 5 628 309
- US-A- 6 019 745
- US-A1- 2002 020 654

## Description

### FIELD OF THE INVENTION

The present invention relates to a cartridge for a medication delivery device. In particular, the present invention relates to a cartridge having electrically conductive layers arranged on surfaces of the cartridge so that these layers form a capacitor in combination. Furthermore, the present invention relates to a method for determining the amount of medicament in the cartridge by capacitive means.

### BACKGROUND OF THE INVENTION

It is known to be a difficult task to determine an amount of medicament remaining in a cartridge for a medication delivery device.

US 5,720,733 discloses an apparatus for electrically determining and recording the dose of an agent delivered with a syringe of the type having a barrel for holding the agent and a plunger for expelling the agent. The syringe includes a capacitive element having first and second conducting surfaces arranged such that the capacitance of the capacitive element varies in dependence upon the dose of agent contained in the barrel. An input terminal and an output terminal located on the outside of the syringe are electrically connected to the first and second conducting surfaces, respectively. The apparatus produces a voltage difference across the terminals, thereby charging the capacitive element. A capacitance meter measure the capacitance and a microprocessor calculates the dose from the measured capacitance. The calculated dose is recorded by a digital memory unit.

US 6,110,148 and US 6,352,523 disclose an apparatus for determining an amount of medication remaining in a syringe. The apparatuses of US 6,110,148 and US 6,352,523 are both equipped with a holder within which the syringe is inserted when the amount of medicament is to be determined. The sidewall of the holder contains various electrodes which can be operated as a capacitor whereby the amount of medicament in the syringe can be determined. US 6,110,148 and US 6,352,523 also disclose a syringe having an integrated capacitor formed between an electrode or electrodes arranged on the housing of the syringe and an electrode arranged on a displaceable piston used to expel medicament from the syringe. In the latter arrangement the capacitance varies in accordance with the axial position of the piston relative to the housing.

US 5,720,733, US 6,110,148 and US 6,352,523 are all concerned with the determination of an amount of agent delivered from a disposable syringe.

WO 2006/021295 relates to a device which is used to determine the filling level of a substance in an ampoule. The device comprises at least two electrodes between which the substance can be introduced. WO 2006/021295 further relates to a method for determining the filling level of a substance in an ampoule comprising at least two electrodes whereby the filling level is determined by measuring the capacity of at least one capacitor formed by at least two electrodes. The dielectric constant of the substance, which is at least partly arranged between two electrodes, influences the capacitive coupling between the two electrodes forming the capacitor.

As depicted in several figures of WO 2006/021295 the provided electrodes are arranged on exterior surface parts of the ampoule. The arrangement of electrodes as suggested in WO 2006/021295 suffers from at least two major disadvantages. Firstly, the provided electrodes are not mechanically protected which makes them vulnerable. Secondly, the capacitor formed by the electrodes is not protected against incoming EMI, whereby measurements of the filling level of the ampoule may easily become inaccurate due to unavoidable electronic noise in the system.

It is an object of the present invention to provide a reliable and easy way of determining the remaining amount of medicament in a replaceable cartridge adapted to be inserted in a medication delivery device.

It is a further object of the present invention to provide a medication delivery device adapted for replaceably receiving a cartridge having a piston slideably arranged inside the cartridge, wherein the device comprises means for continuously or intermittently monitoring the position of the piston during operation of the medication delivery device.

A still further object of the present invention is to provide means for continuously or intermittently monitoring the expelling of a medicament contained in a cartridge during operation of a medication delivery device.

It is an advantage of the present invention that the amount of medicament in the replaceable cartridge may easily be determined.

### SUMMARY OF THE INVENTION

The above-mentioned objects are complied with by providing a cartridge for coupling to a medication delivery device, the cartridge containing a medicament to be expelled from the medication delivery device, the cartridge comprising:
- a body having a sidewall portion being made of an electrically non-conducting material,
- a first electrically conducting layer forming part of or being arranged on a first part of the sidewall portion,
- means for providing an electrical coupling to the medicament contained in the cartridge so that the first electrically conducting layer forms a capacitor with the medicament contained in the cartridge, the medicament forming a virtual capacitor electrode of said capacitor,
wherein an electrically non-conducting intermediate layer is arranged on the first electrically conducting layer, and
wherein an electrically conducting shield layer is arranged on the electrically non-conducting intermediate layer.

The electrically conducting shield layer may be adapted to form an active shield to the first electrically conducting layer. By active shield is meant that an electronic control circuit of an associated medication delivery device ensures that there is essentially no voltage difference between an electrode and its shield electrode counterpart.

The first electrically conducting layer may be arranged on a first exterior surface part of the sidewall portion. The electrode may be made of an optically transparent or non-transparent material. Similarly, the first shield electrode may be made of an optically transparent or non-transparent material. The first electrode and the first shield electrode may be implemented as thin metallic layers, such as thin copper layers.

The first electrically non-conducting layer arranged between electrode and shield electrode may be air in the form of a distance being provided between an electrode and its respective shield electrode. Alternatively, a dielectric layer may be provided between an electrode and its respective shield electrode.

The essentially non-conducting material constituting the sidewall portion of the cartridge may comprise glass or a suitable resin such as polypropylene or a cyclic olefin copolymer (COC).

The overall shape of the electrodes may comprise a variety of shapes, such as quadratic, rectangular, triangular etc. Thus, the first electrically conducting layer may form a first linearly-shaped electrode, said first linearly-shaped electrode being arranged in an axial direction of the body of the cartridge.

The first electrically conducting layer may form a linearly-shaped electrode being arranged in the axial direction of the body of the replaceable cartridge.

The means for providing the electrical coupling to the medicament may comprise an arrangement for establishing a capacitive coupling to the medicament through the sidewall portion of the body of the cartridge. The arrangement for establishing a capacitive coupling may comprise an electrode part disposed at the neck portion of the cartridge.

Alternatively, the means for providing the electrical coupling to the medicament may comprise an electrical connection through a displaceable piston adapted to expel medicament from the cartridge upon its displacement. Alternatively, the means for providing the electrical coupling to the medicament may comprise an electrical connection via an injection needle through which medicament is adapted to be expelled.

### BRIEF DESCRIPTION OF THE DRAWINGS

The present invention will now be explained with reference to the accompanying figures in that
Fig. 1 is a schematic representation of an example showing a general concept for determining the amount of medicament remaining in a cartridge,
Fig. 2 shows a measurement of the capacitance versus cartridge content,
Fig. 3 shows the general concept of a first embodiment of the present invention,
Fig. 4 shows the arrangement of electrodes and shield electrodes on a cartridge,
Fig. 5 shows a first way of establishing an electrical connection to the injection needle,
Fig. 6 shows a second way of establishing an electrical connection to the injection needle
Fig. 7 shows a third way of establishing an electrical connection to the injection needle,
Fig. 8 shows a fourth way of establishing an electrical connection to the injection needle,
Fig. 9 shows a fifth way of establishing an electrical connection to the injection needle,
Fig. 10 shows a sixth way of establishing an electrical connection to the injection needle,
Fig. 11 shows a seventh way of establishing an electrical connection to the injection needle,
Fig. 12 shows an eighth way of establishing an electrical connection to the injection needle, and
Fig. 13 shows a ninth way of establishing an electrical connection to the injection needle.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the scope of the invention as defined by the appended claims.

### DETAILED DESCRIPTION OF THE INVENTION

In its most general aspect the present invention relates to a method and an arrangement for determining the amount of medicament remaining in a cartridge. This amount of medicament is determined by determining the axial position of a displaceable piston positioned in the cartridge. Generally speaking, the concept underlying the present invention includes the determination of a capacitance of an electrical signal path at least comprising one electrode, acting as one of a pair of capacitor plates, and the medicament in the cartridge. In order to determine the capacitance an appropriate time dependent signal is applied to this signal path whereby the capacitance can be determined. As it will become clear later the amount of medicament in the cartridge will strongly influence this capacitance whereby the capacitance becomes an unambiguous measure for the amount of medicament present in the cartridge.

A first example relates to a cartridge having an electrically isolating body of for example glass, a suitable resin, such as polypropylene, or a cyclic olefin copolymer (COC). In order to achieve the advantages of this first example the exterior surface of the plastic/glass body holds a pair of exterior electrodes. This pair of electrodes, which can be made of transparent or non-transparent materials, forms a capacitor in combination.

In this first example the two electrodes are implemented as elongated electrodes arranged on the exterior surface and in the axial direction of the body of the cartridge. Alternatively, the electrodes may be embedded within the wall parts of the cartridge, the embedding ensuring that the electrodes are electrically insulated from the fluid medicament contained in the cartridge. Medicament is expelled from the cartridge by displacing a piston towards a proximal end of the cartridge. As the piston is moved towards the proximal end of the body of the cartridge the spatial overlap between the electrodes and the remaining medicament in the cartridge is decreased causing the capacitance between the two electrodes to decrease as well. Thus, the value of the capacitance between the two electrodes provides a reliable measure for the amount of medicament remaining in the cartridge. Shield electrodes are provided covering the before-mentioned two electrodes in order to obtain a more reliable measurement of the capacitance. Between an electrode and its shield electrode counterpart a non-conducting layer may optionally be provided. Such non-conducting layer may be a dielectric layer. Alternatively, a given electrode and its shield electrode counterpart may be separated by a narrow air gap. Preferably, there is no voltage difference between an electrode and its shield electrode counterpart in that an electronic control circuit ensures that identical signals may be applied to an electrode and its shield electrode counterpart.

In a second example two electrodes integrated with a medication delivery device are used to determine the amount of medicament in a medicament containing cartridge. With a cartridge mounted in the medication delivery device and between the two electrodes the amount of medicament in the cartridge will influence the capacitance of the capacitor formed by the two electrodes and the cartridge with medicament in combination. Thus, a small amount of medicament yields a small capacitance between the two electrodes.

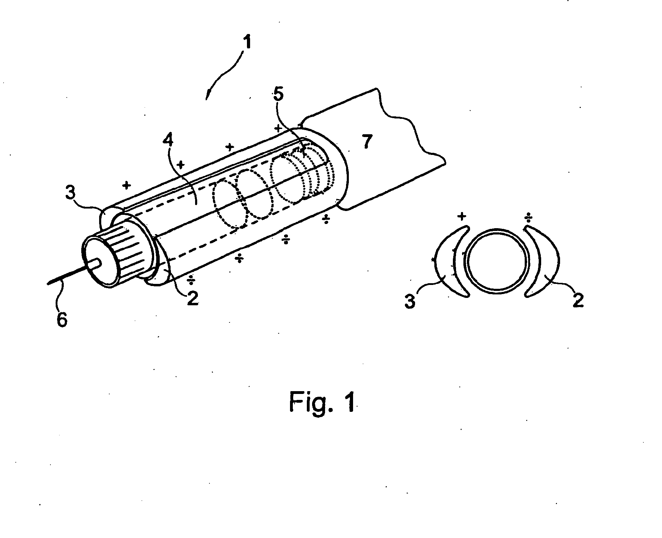

Referring now to Fig. 1 a medication delivery device according to an aspect of the second example is depicted. The medication delivery device 1 comprises two arms 2, 3 between which a medicament containing cartridge 4 is to be mounted. The medicament in the cartridge is expelled by displacing a piston 5 towards the proximal end of the medicament delivery device whereby medicament is expelled through the injection needle 6. The arrangement for displacing the piston towards the proximal end of the medication delivery device is housed within the upper part 7 of the medication delivery device.

Two electrodes forming a capacitor in combination are arranged on the arms 2, 3. The existence of these electrodes is depicted by the plus and minus signs on Fig. 1. The material constituting the body of the cartridge and optionally the medicament that is contained within the body of the cartridge functions as a dielectric between these electrodes so that the capacitance of the capacitor varies in accordance with the volume of the medicament between the electrodes. The electrodes are typically made of copper, but other electrically conducting materials may also be applicable.

In Fig. 1, the arms 2, 3 form part of a mechanism retaining the cartridge in the medication delivery device. The arms 2, 3 may be fixedly arranged with respect to the distal housing part of the medication delivery device. Although not depicted in Fig. 1, the arms 2, 3 may be formed for cooperating with a releasable cartridge retaining cap arranged in the proximal end of the medication delivery device.

Alternatively, the mechanism for holding the cartridge may comprise a separate cartridge holder in the form of an adapter partly of fully encompassing the cartridge, e.g. forming a disposable sub-assembly. In this case, the cartridge holder is provided with the necessary electrodes forming the capacitor electrodes, the electrodes being formed for establishing electrical contact with corresponding pads provided on the housing part that holds remaining electronic circuitry.

A suitable electronic circuit is incorporated in the medication delivery device. This circuit is capable of providing a signal having an appropriate voltage level and an appropriate frequency to the electrodes via a pair of electrical connections. In addition, the electronic circuit is capable of determining the response from the capacitor and to convert the determined capacitance to a measure for the amount of medicament contained in the cartridge positioned between the arms of the medication delivery device. This measure for the amount of medicament in the cartridge is determined from the axial position of the displaceable piston.

In a first embodiment the present invention relates to a cartridge having an electrically isolating body of for example plastic or glass. In order to achieve the advantages of this embodiment an exterior surface part of the plastic/glass body is coated with a thin metallic layer. This layer forms a capacitor with the medicament contained in the cartridge.

When the cartridge is filled or partly filled with a medicament electrical access to the medicament can be provided via an electrically conducting injection needle which penetrates a rubber sealing covering a septum of the cartridge. Alternatively, electrical access to the medicament can be provided via the displaceable piston. A still further alternative may incorporate an electrical conducting member protruding a part of the body of the cartridge for obtaining galvanic contact with the medicament. Such contact may be provided by injection moulding a cartridge having a co-moulded electric conducting member arranged at least at the proximal end of the cartridge, the electric conducting member being electrically isolated from the external metallic layer. Thus, when the cartridge is at least partly filled with a medicament - which is electrically conducting - a signal path is established via the injection needle (or alternatively by said other means) to the medicament. In case electrical access to the medicament is provided via the injection needle, the injection needle can be mounted in a hub which is in a threaded engagement with the cartridge or cartridge holder. To provide electrical access to the injection needle an electrical connection in the form of an integrated wire or other electrical connection is established between the injection needle and the exterior of the hub. When the hub is secured to the cartridge and the cartridge is positioned in the medication delivery device electrical connection is established between the injection needle and an electrical terminal on the medication delivery device. In this way electrical access to the medicament is provided.

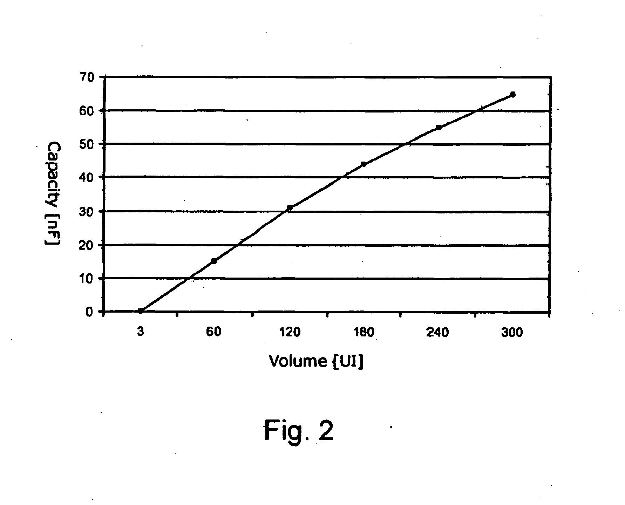

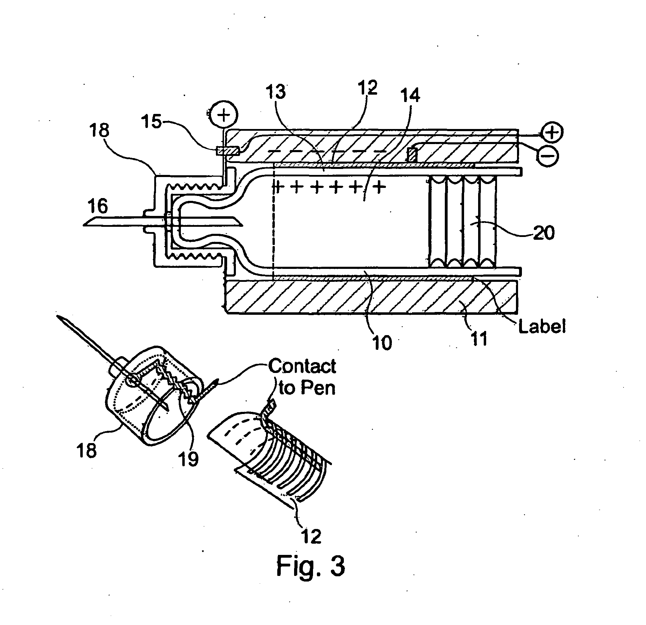

The capacitance between the medicament and the exterior electrode will depend on the amount of medicament in the cartridge. Thus, the amount of medicament in the cartridge can be determined from a measurement of the capacitance between the medicament and the exterior electrode. A measurement of the capacitance versus cartridge content is depicted in Fig. 2 whereas the general concept behind the first embodiment of the present invention is is depicted in Fig. 3. As seen in Fig. 2 the capacitance varies in an approximately linear manner with the amount of medicament (volume) in the cartridge. The volume is measured in units, UI.

Referring now to Fig. 3 a cartridge is positioned in a medication delivery device 11. The cartridge has an outer electrode 12 whereas the medicament 14 in the cartridge forms a virtual inner electrode 13. An electrical path is established from the medicament 14 to a terminal 15 positioned on the medication delivery device 11. The electrical path is established via the medicament 14, the electrically conducting injection needle 16, and an electrical connector 19 arranged in the hub 18 holding the injection needle 16. Thus, via this signal path electrical access to the medicament 14 can be obtained from terminal 15. In this way, the capacitance between the medicament 14 and outer electrode 12 can be measured by applying appropriate signals to the terminal 15 and the outer electrode 12.

The capacitance of the capacitor formed by the medicament 14 and the outer electrode 12 varies in accordance with the volume of the medicament, see Fig. 2. Thus, when the piston 20 is displaced towards the proximal end of the cartridge, the spatial overlap between the medicament 14 and the outer electrode 12 decreases. This causes the capacitance of the capacitor to decrease accordingly.

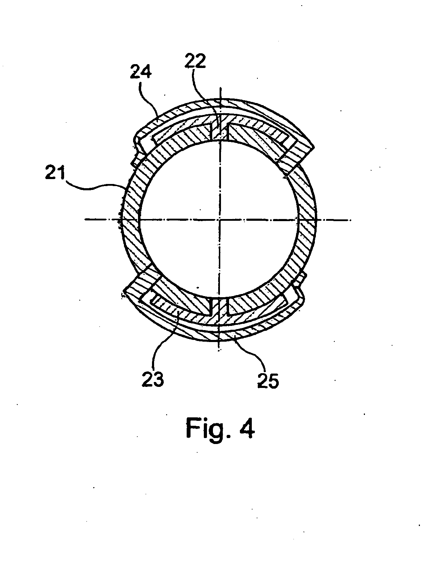

In a preferred embodiment the outer electrode 12 of Fig. 3 is covered with a shield electrode. Referring now to Fig. 4 a cross-sectional view of a cartridge 20 is depicted. As seen in Fig. 4 two electrodes 22, 23 are oppositely arranged. Each electrode is covered by respective shield electrodes 24, 25, said shield electrodes encapsulating the electrodes 22, 23. The shield electrodes 24, 25 extend in essentially the full length of the cartridge. One or more of the shield electrodes may be arranged on the cartridge. Alternatively, one or more of the shield electrodes are formed in a cartridge holder which retains the cartridge. In a medication delivery device which is designed so as to fully encompass the cartridge, the one or more shield electrodes may be arranged in the proximal end of the device. The shield electrodes 24, 25 are preferably operated as active shields. This implies that an electronic control circuit of the medication delivery device ensures that there is essentially no voltage difference between an electrode and its shield electrode counterpart.

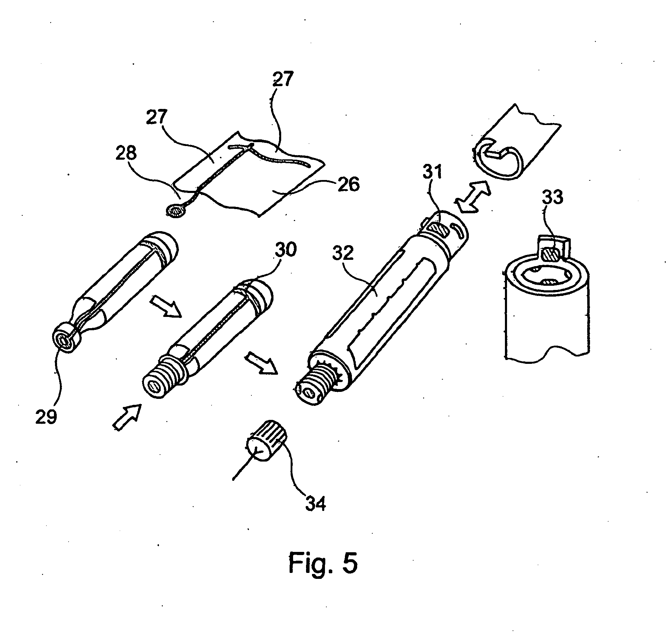

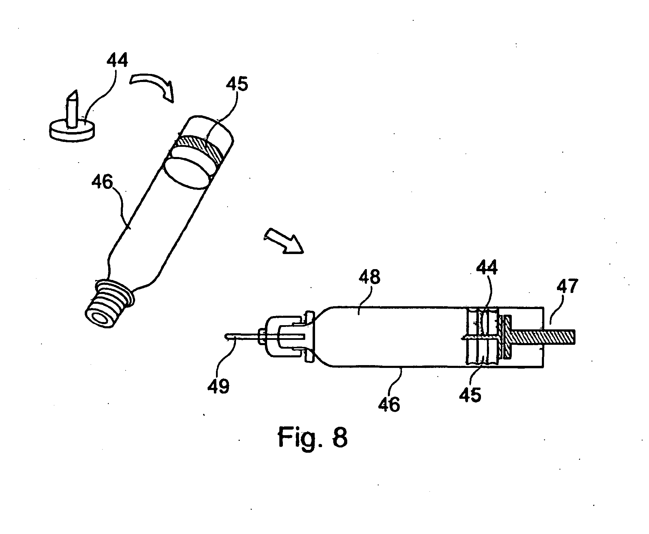

In terms of obtaining an electrical connection to the medicament in the cartridge various implementations are available. With the exception of the example depicted in Fig. 8 the electrical connection to the medicament is provided via an injection needle attached to the medication delivery device. In fig. 5-13, only the electrical conductive structures needed for ensuring galvanically contact with the medicament accommodated in the cartridge are shown. For clarity, the one or more electrodes forming capacitor electrodes arranged on the exterior side of the dielectric wall of the cartridge has been omitted.

Referring now to Fig. 5, a lable 26 with a conducting print 27 added on the same side as the normal printed text is depicted. Alternatively, one or more of the conductive structures are disposed on the opposite side. Thus, when the label is wrapped around the cartridge, the conductive structures may be disposed on the internal face and/or the external face of the label. An extra strip 28 is attached to the normal shaped label with a shape fitting 29 on top of the cartridge septum under the code top. In the other end of the cartridge the shape of the label forms an electrically conducting ring 30 wrapped around the circular surface providing a contact all around the cartridge. A through-going hole 31 on the cartridge holder 32 just above the circular contact surface makes it feasible to establish an electrical connection between the cartridge label 26 and contacts 33 on an associated medication delivery device. The associated medication delivery device comprises an overhanging part including the contact set 33. This contact set 33 is electrically connected to a PCB inside the medication delivery device. An injection needle 34 is attached to the cartridge holder 32 so that a back end (not shown) of the injection needle penetrates the conducting part 29 of the label.

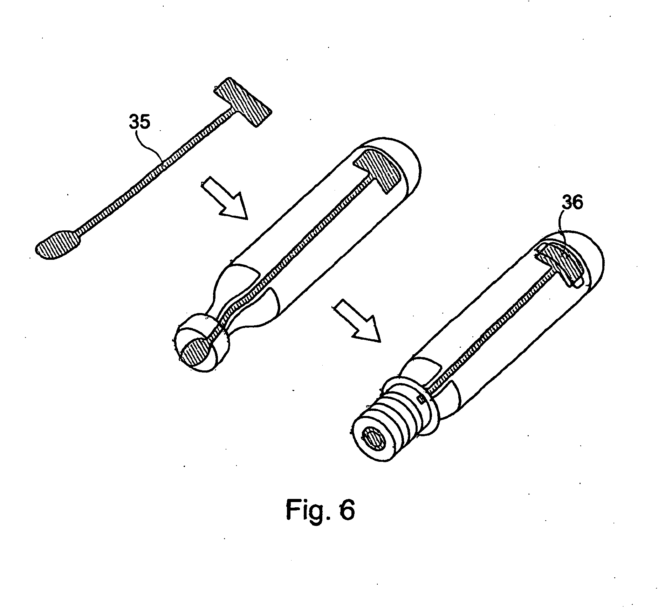

Alternatively, as depicted in Fig. 6, an ordinary label with a conducting metal foil 35 added under the label may be provided. A normal shaped label (44 x 53 mm) is attached to the cartridge. A hole 36 is punched in the label near the end of the cartridge so as to expose the metal foil 35. At the other end of the metal foil 35 the code top is attached on top of the foil between the code top and the septum. An injection needle (not shown) is attached to the cartridge holder so that a back end of the injection needle penetrates the conducting part of the label.

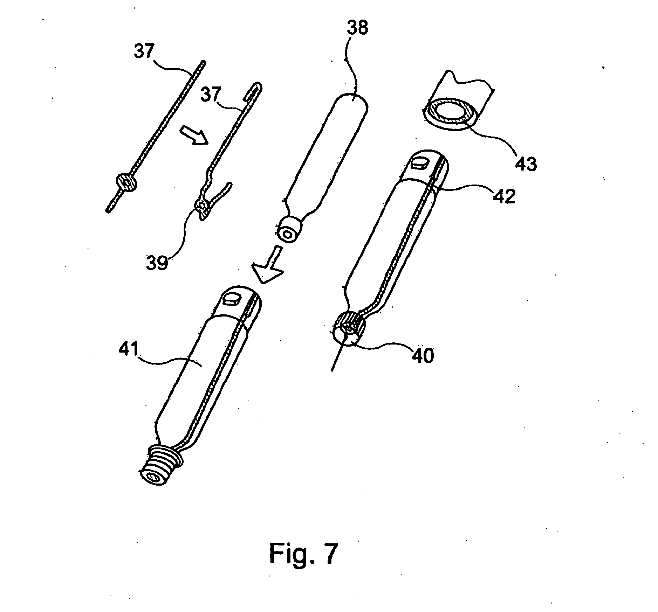

Fig. 7 shows a thin metal spring 37 wrapped around an ordinary cartridge 38 with an integrated contact area 39 on top of the septum connecting direct to the back end of an injection needle 40. The metal spring 37 is mounted together with the cartridge and hidden inside the cartridge holder 41. Before use the contact area 39 is also acting as protection for the septum. In the rear end of the cartridge holder the metal spring 37 is glued and thereby fixed to the cylindrical surface 42. This contact is touching a connection ring 43 in the end of a medication delivery device thereby establishing an electrical connection between the two parts. The contact area 39 may be formed with pre-cut slits to facilitate penetration of the back needle through the contact area 39.

Referring now to Fig. 8, an electrical conducting plug 44 is integrated with or inserted into the plunger 45. When the cartridge 46 is positioned in a medication delivery device a piston rod 47 presses the plunger 45 to its starting position. Along with this, the plug 44 fully penetrates the plunger 45 whereby an electrical connection is established between the medicament 48 contained in the cartridge 46 and an outer portion of the plug 44. An electronic circuit of the medication delivery device is electrically connected to the piston rod 47 whereby an electrical connection is established the medicament 48 accommodated in the cartridge 46.

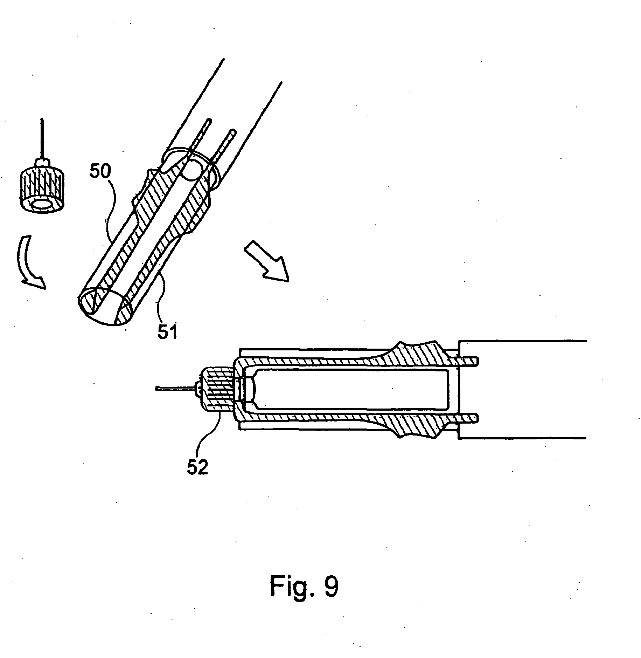

A medication delivery device with a special designed front loaded cartridge holder is shown in Fig. 9. The cartridge holder has two jaws 50, 51 keeping the cartridge in its correct position. The two jaws 50, 51 are electrically conducting and they have a built-in contact switch function. When an injection needle 52 is attached to the medication delivery device the needle contacts to the electronics of the device.

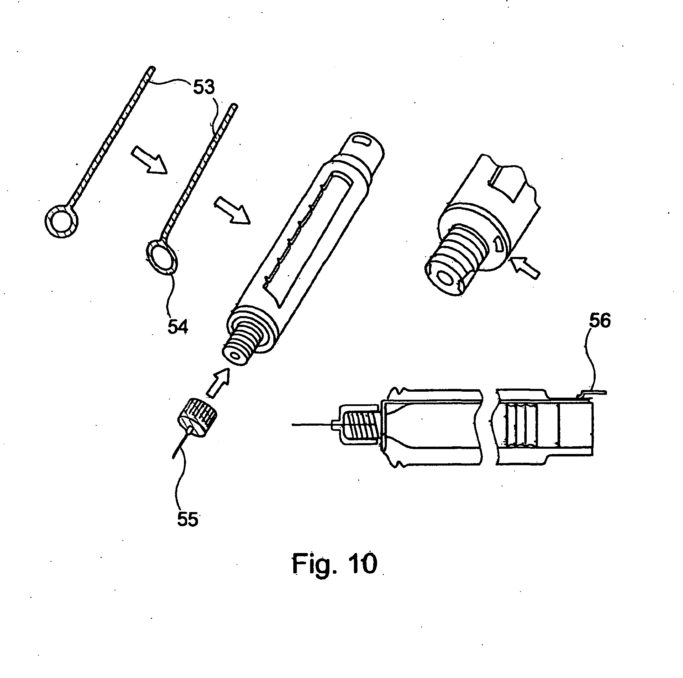

In Fig. 10, a metal spring 53 with a circular contact area 54 is depicted. The metal spring 53 is designed to fit standard cartridges. An injection needle 55 is attached to the device thereby establishing an electrical connection to the metal spring 53. The metal spring 53 has a wire connected to the end of the cartridge holder where a resilient contact set 56 connects to the electronics inside the medication delivery device.

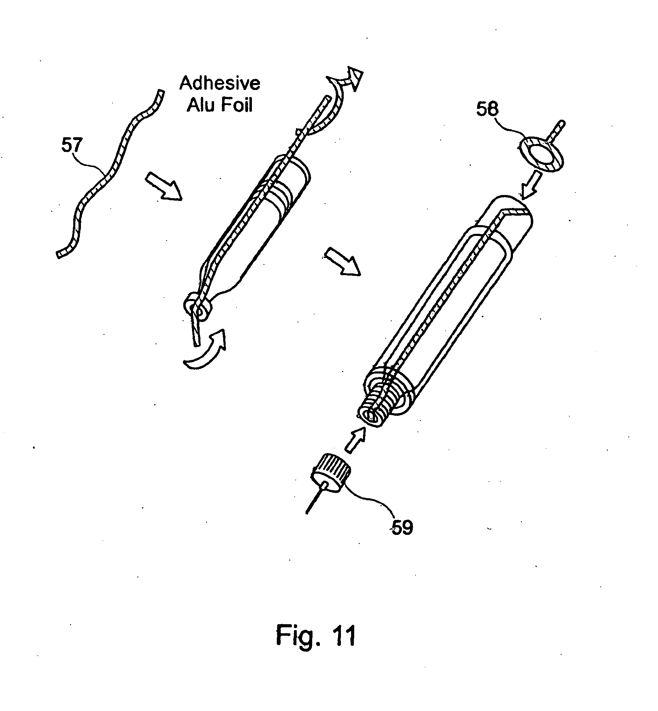

In Fig. 11 a conducting metal foil 57 with adhesive substance is attached on the cartridge. The metal foil extends in the full length of the cartridge and overhangs one end of the cartridge with about 15 mm. A ring shaped conducting steel element 58 connects the metal foil 57 to the electronics of the medication delivery device. An injection needle 59 is attached to the cartridge so that the back end (not shown) of the injection needle penetrates the metal foil attached to the cartridge.

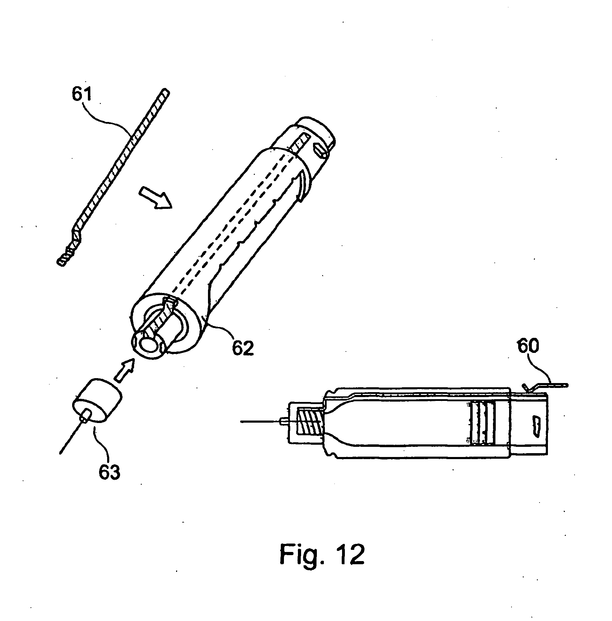

In Fig. 12 a metal spring 60 with a contact area provides electrical contact to a wire 61 attached to the cartridge 62. An injection needle 63 is attached to the medication delivery device whereby an electrical connection is provided between the metal spring 60 and the injection needle 63 via the wire 61. The metal spring 60 is in electrical connection with the electronics of the medication delivery device.

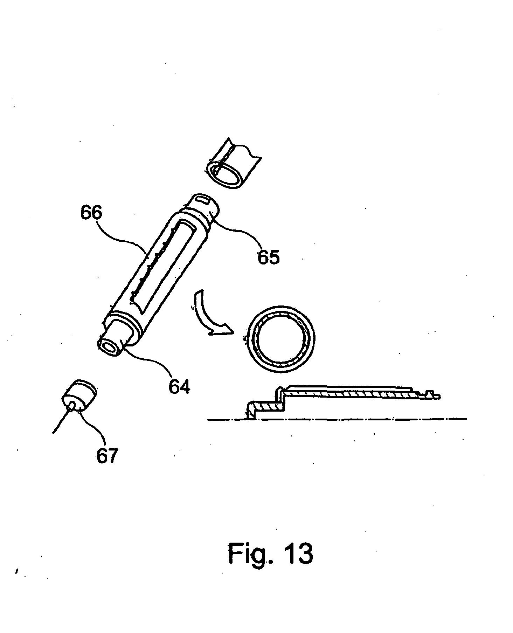

In Fig. 13 a cartridge holder is molded in a two component material where one material is an electrical isolator whereas the other material is an electrical conductor. In regions where electrical connections are to be established, e.g. at the injection needle end 64 and at the connection end 65, the electrically conducting material should be uncovered. Along the cartridge holder body 66 the electrically isolating material covers the electrically conducting material. An injection needle 67 is attached to the cartridge holder.

As mentioned in connection with the first embodiment of the present invention a suitable electronic circuit is incorporated in the medication delivery device. This circuit is capable of providing a signal having an appropriate voltage level and an appropriate frequency. In addition, the electronic circuit is capable of determining the response from the capacitor and to convert the determined capacitance to a measure for the amount of medicament contained in the cartridge.

Beside determining the amount of medicament in the cartridge the present invention may also be applied for other purposes, such as to detect blockage of the injection needle, measuring, in real time, the amount of medicament expelled in a given dose and comparing this measured value with the set dose.

The information obtained by the sensing schemes according to the various embodiments described in this application may be signalled to the user of the injection device for example via a display or may be stored in a memory.

The medication delivery device may further comprise electronic circuitry for calculating and storing amounts of the medicament contained in the cartridge along with previously stored amounts, such as to provide a measure of an expelled dose. The storage of such data may also comprise the corresponding time and date, e.g. determined by the end of an injecting operation. Further, a measure for a given dose expelled by the medication delivery device can be obtained by comparing the current amount of medicament contained in the cartridge with a previously stored amount.

Furthermore, the information obtained by the sensing scheme may be implemented to monitor the instantaneous position of the piston in the cartridge that is the front part of the piston contacting the liquid medicament. Such data may be used for dynamically monitoring the position of the piston during an injecting operation, e.g. for alerting the occurrence of a blocked needle. Also, the position of the piston may be compared to other sensor data obtained in other parts of the injection mechanism for evaluating the fault free operation of the device.

In the depicted embodiments, the electrodes roughly corresponds to the length of a cartridge, or alternatively, at least overlaps the part of the cartridge defined by the distance the piston may be displaced. However, various alternative electrode configurations may be obtained without departing from the scope of this invention. Also, the continuously spanning electrodes may be substituted with a plurality of distinct electrode configurations distributed along the length of the cartridge, each separate electrode having separate connecting leads for connecting to the remaining electronic circuitry of the delivery device.

In addition, parts of exterior electrodes can also be used as reflectors/labels for providing information about the type of medicament contained in a cartridge, whereby associated sensing circuitry can be used for sensing the specific refectors/labels.

## Claims

1. A cartridge for coupling to a medication delivery device, the cartridge containing a medicament to be expelled from the medication delivery device, the cartridge comprising:
- a body (11) having a sidewall portion being made of an electrically non-conducting material,
- a first electrically conducting layer (12) forming part of or being arranged on a first part of the sidewall portion,
- means (28, 29, 30, 35, 36, 37, 39, 44, 53, 54, 57, 61) for providing an electrical coupling to the medicament (14) contained in the cartridge so that the first electrically conducting layer (12) forms a capacitor with the medicament (14) contained in the cartridge, the medicament (14) forming a virtual capacitor electrode (13) of said capacitor,
wherein an electrically non-conducting intermediate layer is arranged on the first electrically conducting layer, and wherein an electrically conducting shield layer is arranged on the electrically non-conducting intermediate layer.

2. A cartridge according to claim 1, wherein the electrically conducting shield layer is adapted to form an active shield to the first electrically conducting layer (12).

3. A cartridge according to claim 1 or 2, wherein first electrically conducting layer (12) forms an outer electrode, the medicament (14) in the cartridge forms an inner electrode (13) and the electrically conducting shield layer forms a shield electrode that covers the outer electrode.

4. A cartridge according to any of claims 1-3, wherein the means (28, 29, 30, 35, 36, 37, 39, 44, 53, 54, 57, 61) for providing the electrical coupling to the medicament (14) comprises an arrangement for establishing a capacitive coupling to the medicament (14) through the sidewall portion of the body.

5. A cartridge according to claim 4, wherein the means (28, 29, 30, 35, 36, 37, 39, 44, 53, 54, 57, 61) for establishing a capacitive coupling comprises an electrode part disposed at the neck portion of the cartridge.

6. A cartridge according to any of claims 1-3, wherein the means (28, 29, 30, 35, 36, 37, 39, 44, 53, 54, 57, 61) for providing the electrical coupling to the medicament (14) comprises an electrical connection (44) through a displaceable piston (45) adapted to expel medicament (14) from the cartridge upon its displacement.

7. A cartridge according to any of claims 1-3, wherein the means (28, 29, 30, 35, 36, 37, 39, 44, 53, 54, 57, 61) for providing the electrical coupling to the medicament (14) comprises an electrical connection (28, 29, 30, 35, 36, 37, 39, 53, 54, 57, 61) adapted to engage an electrical conductive portion of an injection needle (16) through which medicament is adapted to be expelled.

8. A cartridge according to any of claims 1-7, wherein the non-conducting material constituting the sidewall portion of the cartridge comprises plastic or glass.

9. A cartridge according to any of claims 1-8, wherein the first electrically conducting layer (12) forms a linearly-shaped electrode, said linearly-shaped electrode being arranged in an axial direction of the body of the cartridge.

10. A cartridge according to any of claims 1-9, wherein the first electrically conducting layer (12) is made of an optically transparent material.

11. A cartridge according to any of claims 1-10, wherein the electrically conducting shield layer is made of an optically transparent material.

## Patentansprüche

1. Patrone zum Ankoppeln an eine Medikamentenabgabevorrichtung, wobei die Patrone ein aus der Medikamentenabgabevorrichtung auszustoßendes Medikament enthält, wobei die Patrone umfasst:
- einen Körper (11) mit einem aus einem elektrisch nicht leitenden Material hergestellten Seitenwandabschnitt,
- eine erste elektrisch leitende Schicht (12), die einen Teil eines ersten Teils des Seitenwandabschnitts bildet oder daran angeordnet ist,
- ein Mittel (28, 29, 30, 35, 36, 37, 39, 44, 53, 54, 57, 61) zum Bereitstellen einer elektrischen Kopplung an das in der Patrone enthaltene Medikament (14), so dass die erste elektrisch leitende Schicht (12) einen Kondensator mit dem in der Patrone enthaltenen Medikament (14) bildet, wobei das Medikament (14) eine virtuelle Kondensatorelektrode (13) des Kondensators bildet,
wobei eine elektrisch nicht leitende Zwischenschicht an der ersten elektrisch leitenden Schicht angeordnet ist, und wobei eine elektrisch leitende Schutzschicht an der elektrisch nicht leitenden Zwischenschicht angeordnet ist.

2. Patrone nach Anspruch 1, wobei die elektrisch leitende Schutzschicht derart angepasst ist, dass sie für die erste elektrisch leitende Schicht (12) einen aktiven Schutz bildet.

3. Patrone nach Anspruch 1 oder 2, wobei die erste elektrisch leitende Schicht (12) eine Außenelektrode bildet, das Medikament (14) in der Patrone eine Innenelektrode (13) bildet und die elektrisch leitende Schutzschicht eine Schutzelektrode bildet, die die Außenelektrode bedeckt.

4. Patrone nach einem der Ansprüche 1-3, wobei das Mittel (28, 29, 30, 35, 36, 37, 39, 44, 53, 54, 57, 61) zum Bereitstellen der elektrischen Kopplung an das Medikament (14) eine Anordnung zum Einrichten einer Kondensatorkopplung an das Medikament (14) durch den Seitenwandabschnitt des Körpers umfasst.

5. Patrone nach Anspruch 4, wobei das Mittel (28, 29, 30, 35, 36, 37, 39, 44, 53, 54, 57, 61) zum Einrichten einer Kondensatorkopplung ein am Halsteil der Patrone angeordnetes Elektrodenteil umfasst.

6. Patrone nach einem der Ansprüche 1-3, wobei das Mittel (28, 29, 30, 35, 36, 37, 39, 44, 53, 54, 57, 61) zum Bereitstellen der elektrischen Kopplung an das Medikament (14) einen elektrischen Anschluss (44) durch einen verschiebbaren Kolben (45) der zum Ausstoßen des Medikaments (14) aus der Patrone durch dessen Verschiebung angepasst ist, umfasst.

7. Patrone nach einem der Ansprüche 1-3, wobei das Mittel (28, 29, 30, 35, 36, 37, 39, 44, 53, 54, 57, 61) zum Bereitstellen der elektrischen Kopplung an das Medikament (14) einen elektrischen Anschluss (28, 29, 30, 35, 36, 37, 39, 53, 54, 57, 61) umfasst, der zum Eingriff in einen elektrisch leitenden Abschnitt einer Injektionsnadel (16) angepasst ist, durch welche das Medikament zum Ausstoßen angepasst ist.

8. Patrone nach einem der Ansprüche 1-7, wobei das den Seitenwandabschnitt der Patrone bildende Material Kunststoff oder Glas umfasst.

9. Patrone nach einem der Ansprüche 1 bis 8, wobei die erste elektrisch leitende Schicht (12) eine geradförmige Elektrode bildet, wobei die geradförmige Elektrode in axialer Richtung des Körpers der Patrone angeordnet ist.

10. Patrone nach einem der Ansprüche 1-9, wobei die erste elektrisch leitende Schicht (12) aus einem optisch transparenten Material hergestellt ist.

11. Patrone nach einem der Ansprüche 1-10, wobei die elektrisch leitende Schutzschicht aus einem optisch transparenten Material hergestellt ist.

## Revendications

1. Une cartouche pour couplage à un dispositif de délivrance d'une médication, la cartouche contenant un médicament à expulser du dispositif de délivrance de médication, la cartouche comprenant :
- un corps (11) avec une partie de paroi latérale qui est réalisée en un matériau électriquement non conducteur,
- une première couche électriquement conductrice (12) formant une partie ou étant disposée sur une première partie de la région de paroi latérale,
- des moyens (28, 29, 30, 35, 36, 37, 39, 44, 53, 54, 57, 61) pour assurer un couplage électrique au médicament (14) contenu dans la cartouche de sorte que la première couche électriquement conductrice (12) forme un condensateur avec le médicament (14) contenu dans la cartouche, le médicament (14) formant une électrode virtuelle de condensateur (13) dudit condensateur,
dans laquelle une couche intermédiaire électriquement non conductrice est disposée sur la première couche électriquement conductrice, et dans laquelle une couche d'écran électriquement conductrice est disposée sur la couche intermédiaire électriquement non conductrice.

2. Une cartouche selon la revendication 1, dans laquelle la couche d'écran électriquement conductrice est apte à former un écran actif pour la première couche électriquement conductrice (12).

3. Une cartouche selon la revendication 1 ou 2, dans laquelle la première couche électriquement conductrice (12) forme une électrode externe, le médicament (14) dans la cartouche forme une électrode interne (13) et la couche d'écran électriquement conductrice forme une électrode d'écran qui recouvre l'électrode externe.

4. Une cartouche selon l'une des revendications 1 à 3, dans laquelle les moyens (28, 29, 30, 35, 36, 37, 39, 44, 53, 54, 57, 61) pour assurer le couplage électrique au médicament (14) comprennent une configuration pour établir un couplage capacitif au médicament (14) via la région de paroi latérale du corps.

5. Une cartouche selon la revendication 4, dans laquelle les moyens (28, 29, 30, 35, 36, 37, 39, 44, 53, 54, 57, 61) pour établir un couplage capacitif comprennent une partie d'électrode disposée sur la région de col de la cartouche.

6. Une cartouche selon l'une des revendications 1 à 3, dans laquelle les moyens (28, 29, 30, 35, 36, 37, 39, 44, 53, 54, 57, 61) pour assurer le couplage électrique au médicament (14) comprennent une liaison électrique (44) via un piston déplaçable (45) apte à expulser le médicament (14) de la cartouche lorsqu'il se déplace.

7. Une cartouche selon l'une des revendications 1 à 3, dans laquelle les moyens (28, 29, 30, 35, 36, 37, 39, 44, 53, 54, 57, 61) pour assurer le couplage électrique au médicament (14) comprennent une liaison électrique (28, 29, 30, 35, 36, 37, 39, 44, 53, 54, 57, 61) apte à venir en prise avec une région électriquement conductrice d'une aiguille d'injection (16) au travers de laquelle le médicament est apte à être expulsé.

8. Une cartouche selon l'une des revendications 1 à 7, dans laquelle le matériau non conducteur constituant la région de paroi latérale de la cartouche comprend du plastique ou du verre.

9. Une cartouche selon l'une des revendications 1 à 8, dans laquelle la première couche électriquement conductrice (12) forme une électrode de forme linéaire, ladite électrode de forme linéaire étant disposée dans une direction axiale du corps de la cartouche.

10. Une cartouche selon l'une des revendications 1 à 9, dans laquelle la première couche électriquement conductrice (12) est réalisée en un matériau optiquement transparent.

11. Une cartouche selon l'une des revendications 1 à 10, dans laquelle la couche d'écran électriquement conductrice est réalisée en un matériau optiquement transparent.
